# EUROPÄISCHE PATENTANMELDUNG

(11) **EP 0 940 674 A2**
(43) Veröffentlichungstag der Anmeldung: **08.09.1999**
(21) Anmeldenummer: 98124626.7
(22) Anmeldetag: 23.12.1998
(51) Int. Cl.: G01N 27/327, C12Q 1/00, G01N 33/543

(54) **Biosensor mit neuartiger Passivierungsschicht**

(30) Priorität: 18.02.1998 DE 19806642
(71) Anmelder: HÜLS AKTIENGESELLSCHAFT, 45764 Marl (DE)
(72) Erfinder: Kunz, Roland, 45770 Marl (DE); Anders, Christine Dr., 45721 Haltern (DE); Ottersbach, Peter Dr, 51570 Windeck (DE)
(74) Vertreter: Olbricht, Gerhard, Dr.

(57) **Zusammenfassung**

Die Erfindung betrifft einen Biosensor mit einer immobilisierten Biokomponente zur spezifischen Bindung eines Biomoleküls (Analyten), das in einer wäßrigen Matrix vorliegt. Er ist gekennzeichnet durch eine Schicht aus einem organischen Hydrogel als Passivierungsschicht gegen unspezifische Bindungen. Die Erfindung betrifft weiterhin die Verwendung eines organischen Hydrogels als Passivierungsschicht bei der Herstellung von Biosensoren sowie die Verwendung des Biosensors zur Bestimmung eines Biomoleküls (oder Analyten) in einer wäßrigen Matrix, das (bzw. der) von der Biokomponente des Sensors spezifisch gebunden wird.

## Beschreibung

Die Erfindung betrifft einen Biosensor mit einer immobilisierten Biokomponente zur Detektion eines spezifischen Biomoleküls (oder Analyten) in einer wäßrigen Matrix, der eine neuartige, wirkungsvolle Passivierungsschicht gegen unspezifische Bindungen aufweist. Die Erfindung betrifft weiterhin die Verwendung dieser Passivierungsschicht zur Herstellung des Biosensors. Die Erfindung betrifft auch die Verwendung des erfindungsgemäßen Biosensors zur Bestimmung eines Biomoleküls, das von der Biokomponente des Biosensors spezifisch gebunden wird.

### 1. Stand der Technik

Die Funktionsweise von Biosensoren beruht darauf, daß eine Biokomponente, die den Analyten, ein in einer wäßrigen Matrix vorliegendes Biomolekül, spezifisch zu binden vermag, mit einem Transducer (oder Signalwandler) kombiniert ist, der das primäre chemische oder physikalische Signal, das durch die Bindung erzeugt wird, in ein optisches oder elektrisches Sekundärsignal umwandelt, das in üblicher Weise verstärkt und ausgewertet werden kann. Es sind reversibel arbeitende Biosensoren ("Biosensoren" im engeren Sinne) sowie irreversible Biosensoren ("Sonden") bekanntgeworden, die nach einer einmaligen Verwendung verworfen werden. Beispiele für biospezifische, aus der Biokomponente des Sensors und dem Biomolekül als Analyt bestehende biologische Bindungspaare sind Antikörper und Antigen, Rezeptor und Ligand sowie Enzym und Substrat. Bei modernen Biosensoren ist die Biokomponente auf der Oberfläche des Transducers immobilisiert.

Der Transducer kann beispielsweise ein piezoelektrischer Kristall sein, der auf die Masseänderung auf seiner Oberfläche infolge der spezifischen Bindung des Biomoleküls mit einer Veränderung seines Schwingungsverhaltens reagiert, die die Tatsache der Bindung (qualitativ) und gegebenenfalls deren Ausmaß (quantitativ) anzeigt. Anstatt die piezoelektrischen Schwingungen für den Signalumwandlungsprozeß zu nutzen, kann man auch die akustischen Oberflächenwellen (surface acoustic waves; SAW) von piezoelektrischen Substraten dafür verwenden. Die Eindringtiefe der akustischen Oberflächenwelle liegt im Bereich der Wellenlänge, so daß auf der Oberfläche spezifisch gebundene Biomoleküle die Wellenübertragung beeinflussen. Die Biosensoren, die mit Transducern auf der Grundlage der piezoelektrischen Schwingungen oder der akustischen Oberflächenwellen arbeiten, sind Massesensoren, denn sie sprechen letztendlich auf die Veränderung der Masse auf der Oberfläche des Transducers an. Ein anderer bekannter Typ von Biosensoren benutzt Transducer, die die empfangenen Signale in optische Parameter umsetzen und Veränderungen der Strahlungsabsorption, der Emission nach Anregung der Probe, der Reflexion, Brechung und Beugung oder der Polarisation zur Bestimmung des Analyten ausnutzen.

Die vom Transducer abgegebenen Signale werden elektronisch verstärkt und vergleichend ausgewertet. Als Ergebnis erhält man eine Ja/Nein-Aussage oder, bei der quantitativen Analyse, einen Zahlenwert.

Die Empfindlichkeit des Nachweises eines Biomoleküls in einer Matrix mit einem Biosensor wird dadurch herabgemindert, daß der Transducer nicht nur die Signale, die von der spezifischen Bindung des Biomoleküls an die Biokomponente des Sensors herrühren, in elektrische oder optische Signale umwandelt, sondern auch Signale, die von einer unspezifischen Bindung anderer Komponenten der Matrix stammen. Diese unspezifischen Bindungen können adsorptiv, absorptiv, kovalent oder ionischer Natur sein und verfälschen das Analysenergebnis. Das vom Transducer zur Auswertung weitergeleitete Signal setzt sich also additiv aus dem Signal der spezifischen und dem der unspezifischen Bindung zusammen. Man könnte den durch das unspezifische Signal verursachten Fehler tolerieren, wenn er bei einer bestimmten analytischen Aufgabe in einer gleichen oder zumindest gleichartigen Matrix konstant wäre. Das ist jedoch nicht der Fall. So ist z.B. das Ausmaß der unspezifischen Bindung bei der Bestimmung von Antigenen in Blut von Spender zu Spender unterschiedlich. Man erhält also selbst bei Abwesenheit des Antigens Werte mit einer Streuung, deren Ausmaß durch die Standardabweichung von dem Mittelwert der Blindmessungen charakterisiert werden kann. Je größer die Standardabweichung, umso geringer ist die Nachweisempfindlichkeit, denn ein Test wird üblicherweise erst dann als positiv gewertet, wenn der Meßwert das Dreifache der Standardabweichung übersteigt.

Es ist daher erwünscht, die unspezifischen Bindungen zu verhindern oder zumindest zurückzudrängen, um die Standardabweichung zu minimieren. Bei einer bekannten Variante (siehe z.B. K.A.Davis. T.R. Leary; Continuous Liquid Phase Piezoelectric Biosensor for Kinetic Immunoassays, Analy. Chem. 61 (1989), 1227-1230) drängt man mit Rinderserumalbumin (BSA) als Passivierungsschicht die unspezifische Bindung von Eiweißkörpern bei der Bestimmung von Immunoglobulin G (IgG) in Phosphat-gepufferter Natriumchloridlösung (PBS) zurück. Die Wirksamkeit dieser Passivierungsschicht ist allerdings nicht optimal, weil einerseits die Makromoleküle des BSA die Oberfläche des Transducers nicht hermetisch abdecken, so daß unbesetzte Stellen für unspezifische Bindungen verbleiben, und andererseits BSA seinerseits hydrophobe Gruppen, z.B. in Fettsäuremolekülen. unspezifisch bindet.

### 2. Kurzbeschreibung der Erfindung

Es wurde nun gefunden, daß organische Hydrogele Passivierungsschichten für Biosensoren ergeben, die gegen unspezifische Bindungen hervorragend wirksam sind. Ein Gegenstand der Erfindung ist dementsprechend ein Biosensor mit einer immobilisierten Biokomponente zur spezifischen Bindung eines Biomoleküls (Analyten), das in einer wäßrigen Matrix vorliegt. Dieser Biosensor weist als wesentliches Merkmal eine Schicht aus einem organischen Hydrogel als Passivierungsschicht gegen unspezifische Bindungen auf.

Ein anderer Gegenstand der Erfindung ist die Verwendung eines organischen Hydrogels als Passivierungsschicht bei der Herstellung von Biosensoren.

Ein weiterer Gegenstand der Erfindung ist die Verwendung des erfindungsgemäßen Biosensors zur Bestimmung eines Biomoleküls (oder Analyten) in einer wäßrigen Matrix, das (bzw. der) von der Biokomponente des Sensors spezifisch gebunden wird.

### 3. Vorteile der Erfindung

Die erfindungsgemäßen Biosensoren zeichnen sich durch eine erheblich kleinere Standardabweichung von dem Mittelwert der Blindmessungen und damit durch eine größere Empfindlichkeit gegenüber bekannten Biosensoren mit oder ohne Passivierungsschicht aus. Das liegt möglicherweise an der Hydrathülle der Hydrogele, die einer Annäherung und unspezifischen Bindung solcher Moleküle entgegenwirkt, die von dem Analyten verschieden sind. Die Passivierungsschicht haftet besonders fest, und die Biosensoren eignen sich daher auch besonders als reversible Sensoren, wenn das Hydrogel nach gut bekannten Techniken durch Pfropfpolymerisation aufgebracht wird. Ein weiterer Vorteil besteht darin, daß die Querempfindlichkeit verringert (oder die Spezifität für den gesuchten Analyten erhöht) wird.

### 4. Beschreibung der Erfindung

Die Passivierungsschicht aus einem organischen Hydrogel ist das wesentliche Merkmal des erfindungsgemäßen Biosensors. Hinsichtlich aller anderen Gegebenheiten entspricht er den Sensoren nach dem Stand der Technik. Er ist vorteilhaft ein Massesensor und enthält als Biokomponente in der Regel einen Antikörper, ein Enzym oder einen Rezeptor, vorteilhaft ionisch oder kovalent unmittelbar oder zweckmäßig über eine polymere Zwischenschicht auf dem Transducer gebunden. Der Transducer kann aus den für diesen Zweck bekannten, üblichen Materialien bestehen, z.B. aus Siliciumnitrid (Si₃N₄), Siliciumdioxid oder Lanthanniobat (LaNbO₃). Ebenso bietet der Biosensor nach der Erfindung hinsichtlich der Verarbeitung und Auswertung des im Transducer erzeugten Sekundärsignals keine Besonderheiten.

Bei einer bevorzugten Ausführungsform des erfindungsgemäßen Biosensors ist die Passivierungsschicht aus dem organischen Hydrogel, unmittelbar oder vorzugsweise mittelbar über eine Zwischenschicht, auf die Oberfläche des Transducers aufgebracht, zweckmäßig durch Pfropfung der hydrophilen Monomeren, und enthält auf seiner freien Oberfläche kovalent oder ionisch gebunden oder in das Hydrogel integriert die Biokomponente zur spezifischen Bindung des Biomoleküls in der Matrix. Biosensoren mit einer Passivierungsschicht, die auf eine aktivierte polymere Zwischenschicht gepfropft wurde und auf ihrer freien Oberfläche die Biokomponenten ionisch oder kovalent gebunden enthält, zeichnen sich durch eine besonders geringe Querempfindlichkeit aus.

### 4.1 Passivierungsschicht / Hydrophile Vinylmonomere

Das organische Hydrogel der Passivierungsschicht entsteht durch Polymerisation, gegebenenfalls Pfropfpolymerisation, von hydrophilen Vinylmonomeren, gegebenenfalls zusammen mit Anteilen von hydrophoben Vinylmonomeren. Zum Hydrogel wird das hydrophile Polymer bei Kontakt mit einem wäßrigen Medium, eine Bedingung, die bei bestimmungsgemäßem Gebrauch immer gegeben ist. Die Herstellung von Hydrogele bzw. von Polymeren, die ein Hydrogel ergeben, ist in den deutschen Patentanmeldungen 197 00 079.7 (O.Z. 5146), 197 15 449.2 (O.Z. 5176) und 197 27 556.7 (O.Z. 5210) beschrieben.

Das Hydrogel der Passivierungsschicht ist natürlich seinerseits hydrophil. Als Passivierungsschicht bevorzugt werden Hydrogele, die bei der Bestimmung des Kontaktwinkels einer Luftblase nach der Vorschrift von R.J.Good et al., Techniques of Measuring Contact Angles in Surface and Colloid Science (Hrsg. R.J.Good) Vol. 11. Plenum Press, New York, N.Y. einen Kontaktwinkel <40°, vorteilhaft <30° zeigen. Dabei läßt man unterhalb der von Wasser umgebenen Probe eie Luftblase, die die Oberfläche je nach deren Hydrophilität mehr oder minder stark benetzt.

Geeignete hydrophile Vinylmonomere enthalten mindestens eine olefinische Doppelbindung sowie mindestens eine hydrophile Gruppe. Die olefinischen Doppelbindungen können in verschiedenartigen funktionalen Resten vorliegen, beispielsweise in Alkenylresten, wie Vinyl- oder Allylresten, oder in Resten, die sich von ungesättigten Carbonsäuren oder deren Derivaten ableiten, wie Acrylsäure, Methacrylsäure, den Amiden dieser Carbonsäuren oder Maleinsäure. Auch hinsichtlich der hydrophilen Gruppen herrscht große Mannigfaltigkeit. Von den geeigneten hydrophilen Gruppen seien beispielsweise genannt: Hydroxylgruppen, Ethergruppen, Acyloxygruppen, Carboxylgruppen, Carbonsäureestergruppen, Carbonsäureamidgruppen, Carboalkoxygruppen und Nitrilgruppen; 1,2-Epoxidgruppen; Schwefelsäureester, Sulfonsäure, Sulfinsäure-, Phosphorsäure-, Phosphonsäure- und Phosphinsäuregruppen einschließlich ihrer entsprechenden Salze und Ester; primäre, sekundäre und tertiäre Aminogruppen; Acylaminogruppen, die offenkettig oder in einen Ring eingebaut sein können; Polyalkylenoxidgruppen, wie Polyethylenoxidgruppen und Polypropylenoxidgruppen mit oder ohne endständige Hydroxylgruppe; Polyester-, Polyesteramid- und Polyetheresteramidgruppen; der Pyrrolidonring und ähnliche Heterocyclen sowie Reste von olefinisch funktionalisierten Zuckern. Natürlich kommt es für die Hydrophilität eines Monomeren auf die Balance zwischen hydrophilen und hydrophoben Anteilen im Molekül des Monomeren an. Für die Erfindung geeignete Monomere sind bei 20°C in Wasser zu mindestens 1 Gewichtsprozent, vorteilhaft zu mindestens 10 Gewichtsprozent und insbesondere zu mindestens 40 Gewichtsprozent löslich, jeweils bezogen auf die gesamte Lösung.

Die für die Erfindung verwendeten hydrophilen Vinylmonomeren enthalten vorzugsweise eine olefinische Doppelbindung und eine hydrophile Gruppe. Sie können aber auch mehrere olefinische Doppelbindungen und/oder hydrophile Gruppen aufweisen. So sind z.B. offenkettige Polyalkylenoxide mit zwei endständigen Vinyl-, Allyl-, Acryloxy- oder Methacryloxy-Gruppen gut geeignet.

Von den geeigneten hydrophilen Vinylmonomeren seien beispielsweise genannt: Acrylsäure und deren Derivate, z.B. Acrylamid, N,N,-Dimethylacrylamid, Acrylnitril, Methylacrylat, 2-Hydroxyethylacrylat, 2-Hydroxypropylacrylat, 2-Methoxyethylacrylat; 2-Ethoxyethylacrylat, 4-Hydroxybutylacrylat und 1,4-Butandioldiacrylat, sowie Methacrylsäure und deren entsprechende Derivate; Carbonsäurevinylderivate, wie Vinylacetat, N-Vinylacetamid und N-Vinylpyrrolidon; Vinylsulfonsäuren und deren Alkalisalze, wie Natriumvinylsulfonat; Alkenylarylsulfonsäuren und deren Alkalisalze, wie Styrolsulfonsäure und Natriumstyrolsulfonat, Vinylether, wie Vinylmethylether, Vinylethylether, Vinylglycidylether, Diethylenglykoldivinylether und Vinyl-n-butylether; Vinylketone, wie Vinylmethylketon, Vinylethylketon und Vinyl-n-propylketon; Vinylamine, wie N-Vinylpyrrolidin; Polyalkylenoxyverbindungen mit endständigen Allyl-, Vinyl-, Acryl- oder Methacrylgruppen, wie Ethoxytetraethoxyethylacrylat oder -methacrylat, n-Propoxydodecaethylenoxyethylvinylether, Polyethylenglykolmono- oder diacrylate mit Molgewichten von 600 oder 1.200 Poly-(ethylen/propylen)glykolmono- oder dimethacrylate mit Molgewichten von 400 und 800 sowie Allyloxyoctapropylenoxyethanol; Zuckerderivate, wie vinylsubstituierte Arabinose oder acryloylierte Hydroxypropylzellulose; und funktionalisierte Polyalkylenglykole, wie Triethylenglykoldiacrylat oder Tetraethylenglykoldiallylether.

Wenn man tri- oder höherfunktionelle Vinylmonomere mitverwendet, erhält man ein vernetztes Copolymer, wodurch man, insbesondere bei der in der Folge beschriebenen Pfropfpolymerisation, eine besonders dichte Abdeckung der Oberfläche des Transducers und damit eine größere Meßgenauigkeit des Biosensors erreicht. Geeignete Tri- oder höherfunktionelle Vinylmonomere sind u.a. Polyacrylate, wie Trimethylolpropantriacrylat, Trimethylolpropantrimethacrylat und Pentaerythrittetraacrylat; Polyvinylether, wie Glycerin-12EO-trivinylether und Pentarythrittetraallylether. Die vernetzenden Vinylmonomeren werden gegebenenfalls in Mengen eingesetzt, die zweckmäßig 0.01 bis 1 mol-%. bezogen auf die gesamten Monomeren, betragen.

### 4.2 Hydrophobe Vinylmonomere als Comonomere

Neben den hydrophilen Monomeren können bei der Herstellung der Hydrogele bzw. der Polymeren, die bei Kontakt mit einem wäßrigen Medium ein Hydrogel sind, gewissen Mengen, z.B. bis zu 60 mol-%, bezogen auf die gesamten Monomeren, an hydrophoben Vinylmonomeren mitverwendet werden, z.B. α-Olefine, wie Propen, 1-Buten und 1-Octen; Vinylchlorid; Vinylaromaten, wie Styrol, α-Methylstyrol und Vinyltoluol.

An Stelle der erwähnten gegebenenfalls mitzuverwendenden vernetzenden hydrophilen Vinylmonomeren kann man auch tri- oder höherfunktionelle hydrophobe Vinylmonomere in den genannten Mengen mitverwenden und erzielt dann im wesentlichen die gleiche beschriebene Wirkung.

Der molare Anteil der hydrophoben Vinylmonomeren sollte so bemessen sein, daß das Hydrogel in dem oben beschriebenen Test nach R.J.Good et al, einen Kontaktwinkel von <40°, vorzugsweise von <30° zeigt.

### 4.3 Herstellung der organischen Hydrogele im Bulk

Die Monomeren können, jeweils einzeln oder aber als dem jeweiligen Verwendungszweck angepaßtes Gemisch, in üblicher Weise polymerisiert werden, zweckmäßig durch radikalisch initiierte Lösungs- oder Emulsionspolymerisation. Die Monomeren werden im allgemeinen als 1-bis 40-, vorteilhaft als 5- bis 20-gewichtsprozentige Lösungen eingesetzt. Das Lösemittel ist zweckmäßig Wasser. Das erhaltene Polymer ist dann unmittelbar ein Hydrogel. Arbeitet man mit einem organischen Lösemittel, so wird das Polymer spätestens bei der Verwendung des Biosensors im Kontakt mit der wäßrigen Matrix zum Hydrogel. Man verwendet übliche Polymerisationsinitiatoren, z.B. organische Peroxyverbindungen oder Hydroperoxide, Azoverbindungen, Persäuren, Persulfate oder Percarbonate, in den üblichen Mengen und löst die Polymerisation thermisch oder durch Bestrahlung aus. Die Lösungen oder Emulsionen sind unmittelbar zur Beschichtung der Transducer geeignet. Vorteilhaft geschieht dies durch Spin-Coating, wodurch man Passivierungsschichten aus dem Polymer mit Schichtdicken von 0,1 bis 100 µm erzeugen kann.

Alternativ kann man die Passivierungsschicht durch Pfropfung des vorgebildeten Hydrogel-Polymers auf die Oberfläche des Transducers erzeugen. In diesem Falle bringt man zweckmäßig vor der Pfropfung eine Zwischenschicht aus einem (Co)polymer auf, die so aktiviert ist oder werden kann, daß die Pfropfung möglich ist. Die Pfropfung wird bevorzugt, weil sie zu einer fest haftenden, besonders dünnen und trotzdem dichten Beschichtung der Oberfläche des Transducers führt.

### 4.4 Polymere Zwischenschicht und deren Aktivierung

Für die Zwischenschicht eignen sich Standardpolymere mit oder ohne strahlungssensitive(n) Gruppen, wie Polyurethane, Polyamide, Polyester und Polyether, Polyether- oder -esterblockamide, Polysiloxane, Polystyrol, Polyvinylchlorid, Polycarbonate, Polyolefine, Polysulfone Polyisopren, Polychloropren, Polytetrafluorethylen, Polyacrylate, Polymethacrylate sowie entsprechende Copolymere oder Blends. Die Zwischenschicht wird wiederum zweckmäßig durch Spin-Coating aus organischen Lösungen aufgebracht und ist im allgemeinen ebenfalls 0,01 bis 100 µm stark.

Die Oberfläche der Zwischenschicht kann zur Vorbereitung der Pfropfung nach einer Reihe von Methoden aktiviert werden, von denen die wichtigsten genannt seien.
(1) Die Aktivierung von Standardpolymeren ohne UV-strahlungssensitive Gruppen kann vorteilhaft durch UV-Strahlung, z.B. im Wellenlängenbereich von 100 bis 400 nm, vorzugsweise von 125 bis 310 nm erfolgen. Besonders gute Ergebnisse wurden mit weitgehend monochromatischer, kontinuierlicher Strahlung erzielt, wie sie z.B. von Excimer-UV-Strahlern (Fa. Heraeus, Kleinostheim, BR Deutschland) beispielsweise mit F₂, Xe₂, ArF, XeCl, KrCl und KrF als Lampenmedium, erzeugt wird. Aber auch andere Strahlungsquellen, wie Quecksilberdampflampen mit breitem Strahlungsspektrum und Strahlungsanteilen im sichtbaren Bereich, sind geeignet, sofern sie erhebliche Strahlungsanteile in den genannten Wellenlängenbereichen emittieren. Es hat sich gezeigt, daß die Anwesenheit kleiner Mengen an Sauerstoff vorteilhaft ist. Die bevorzugten Sauerstoffpartialdrücke liegen zwischen 2x10⁻⁵ und 2x10⁻² bar. Man arbeitet beispielsweise in einem Vakuum von 10⁻⁴ bis 10⁻¹ bar oder unter Verwendung eines Inertgases, wie Helium, Stickstoff oder Argon, mit einem Sauerstoffgehalt von 0.02 bis 20 Promille. Die optimale Bestrahlungsdauer hängt von dem polymeren Substrat, der Zusammensetzung des umgebenden Gasmediums, der Wellenlänge der Strahlen sowie der Leistung der Strahlenquelle ab und läßt sich durch orientierende Vorversuche unschwer ermitteln. Im allgemeinen bestrahlt man die Substrate 0,1 Sekunde bis 20 Minuten lang, insbesondere 1 Sekunde bis 10 Minuten. Bei diesen kurzen Bestrahlungszeiten erwärmt sich das polymere Substrat nur in geringem Maße, und es treten selbst bei Strahlen mit Wellenlängen am harten Ende des genannten weiteren Bereichs keine unerwünschten Nebenreaktionen auf, die zu Schäden an den exponierten Oberflächen führen könnten.
(2) Die Aktivierung kann erfindungsgemäß auch durch ein Hochfrequenz- oder Mikrowellenplasma (Hexagon, Fa. Technics Plasma, 85551 Kirchheim, Deutschland) in Luft, Stickstoff- oder Argonatmosphäre erreicht werden. Die Expositionszeiten betragen im allgemeinen 30 Sekunden bis 30 Minuten, vorzugsweise 2 bis 10 Minuten. Der Energieeintrag liegt bei Laborgeräten zwischen 100 und 500W, vorzugsweise zwischen 200 und 300W.
(3) Weiterhin lassen sich auch Korona-Geräte (Fa. SOFTAL. Hamburg, Deutschland) zur Aktivierung verwenden. Die Expositionszeiten betragen in diesem Falle in der Regel 1 Sekunde bis 10 Minuten, vorzugsweise 1 bis 60 Sekunden.
(4) Die Aktivierung durch Elektronen- oder gamma-Strahlen (z.B. aus einer Kobalt-60-Quelle) ermöglicht kurze Expositionszeiten, die im allgemeinen 0,1 bis 60 Sekunden betragen.
(5) Beflammungen von Oberflächen führen ebenfalls zu deren Aktivierung. Geeignete Geräte, insbesondere solche mit einer Barriere-Flammenfront, lassen sich auf einfache Weise bauen oder beispielsweise beziehen von der Fa. ARCOTEC, 71297 Mönsheim, Deutschland. Sie können mit Kohlenwasserstoffen oder Wasserstoff als Brenngas betrieben werden. In jedem Fall muß eine schädliche Überhitzung der Zwischenschicht vermieden werden, was durch innigen Kontakt mit einer gekühlten Metallfläche auf der von der Beflammungsseite abgewandten Oberfläche leicht erreicht wird. Die Expositionszeiten belaufen sich im allgemeinen aus 0,1 Sekunde bis 1 Minute, vorzugsweise 0,5 bis 2 Sekunden, wobei es sich ausnahmslos um nicht leuchtende Flammen handelt und die Abstände der Oberfläche der Zwischenschicht zur äußeren Flammenfront 0,2 bis 5 cm, vorzugsweise 0,5 bis 2 cm betragen.
(6) Weiterhin lassen sich die Oberflächen der Zwischenschichten auch durch Behandlung mit starken Säuren oder Basen aktivieren. Von den geeigneten starken Säuren seien Schwefelsäure, Salpetersäure und Salzsäure genannt. Man kann z.B. Polyamide 5 Sekunden bis 1 Minute mit konzentrierter Schwefelsäure bei Raumtemperatur behandeln. Als starke Basen eignen sich besonders Alkalimetallhydroxide in Wasser oder einem organischen Lösemittel. So kann man z.B. verdünnte Natronlauge 1 bis 60 Minuten bei 20 bis 80°C auf die Substrate einwirken lassen. Alternativ können z.B. Polyamide aktiviert werden, indem man 2 %-iges KOH in Tetrahydrofuran 1 Minute bis 30 Minuten auf die Substratoberfläche einwirken läßt.
(7) Schließlich können schon bei der Herstellung der Polymeren für die Zwischenschicht Monomere mit UV-strahlungssensitiven Gruppen einpolymerisiert werden. Als solche eignen sich u.a. Furyl- oder Cinnamoylderivate, die z.B. in Mengen von 3 bis 15 mol% angewandt werden können. Gut geeignete Monomere dieser Art sind Cinnamoylethylacrylat und -methacrylat.

In manchen Fällen, z.B. bei hochhydrophoben Polymeren, kann es empfehlenswert sein, die Oberflächen der Zwischenschicht durch eine Kombination aus zwei oder mehr der genannten Methoden zu aktivieren. Die bevorzugte Aktivierungsmethode ist die mit UV-Strahlung gemäß Ziffer (1).

### 4.5 Herstellung der Passivierungsschicht durch Pfropf(co)polymerisation von Monomeren

Alternativ kann man die Passivierungsschicht auch durch Propf(co)polymerisation erzeugen, indem man die Monomeren auf die aktivierte Zwischenschicht pfropft. Wenn die Zwischenschichten nach einer der unter (1) bis (6) beschriebenen Methoden aktiviert wurden, werden die aktivierten Oberflächen zweckmäßig 1 bis 20 Minuten, vorzugsweise 1 bis 5 Minuten der Einwirkung von Sauerstoff, z.B. in Form von Luft, ausgesetzt.

Anschließend werden die (gegebenenfalls auch gemäß (7)) aktivierten Oberflächen der Zwischenschichten nach bekannten Methoden, wie Tauchen, Sprühen oder Streichen, mit Lösungen des oder der erfindungsgemäß zu verwendenden Vinylmonomeren beschichtet. Als Lösemittel haben sich Wasser-Ethanol-Gemische bewährt, doch sind auch andere Lösemittel verwendbar, sofern sie ein ausreichendes Lösevermögen für das Monomer oder die Monomeren besitzen und die Oberflächen gut benetzen. Je nach Löslichkeit der Monomeren und gewünschter Schichtdicke der fertigen Beschichtung können die Konzentrationen der Monomeren in der Lösung 1 bis 40 Gew.-% betragen. Lösungen mit Monomerengehalten von 5 bis 20 Gew.-%, beispielsweise von etwa 10 Gew.-%, haben sich in der Praxis bewährt und ergeben im allgemeinen in einem Durchgang zusammenhängende, die Oberfläche der Zwischenschichten bedeckende Beschichtungen mit Schichtdicken, die mehr als 0,1 µm betragen können.

Nach dem Verdampfen des Lösemittels oder während des Verdampfens wird die Polymerisation oder Copolymerisation des oder der auf die aktivierte Oberfläche aufgebrachten Monomeren zweckmäßig durch Strahlen im kurzwelligen Segment des sichtbaren Bereiches oder im langwelligen UV-Bereich der elektromagnetischen Strahlung induziert. Gut geeignet ist z.B. die Strahlung mit Wellenlängen von 250 bis 500 nm, vorzugsweise von 290 bis 320 nm. Strahlen im genannten Wellenlängenbereich sind relativ weich, selektiv bezüglich der Polymerisation und greifen die polymere Zwischenschicht nicht an. Wie bei der Aktivierung der Oberflächen der Zwichenschicht ist es auch hier vorteilhaft, mit einer Strahlenquelle zu arbeiten, die weitgehend monochromatische, kontinuierliche Strahlen emittiert. Besonders geeignet sind wiederum Excimer-UV-Strahler mit kontinuierlicher Strahlung, z.B. mit XeCl oder XeF als Strahlermedium. Die erforderliche Intensität der Strahlen und die Dauer der Einwirkung hängen von den jeweiligen hydrophilen Monomeren ab und lassen sich durch orientierende Versuche ohne weiteres ermitteln. Im Prinzip sind auch hier Quecksilberdampflampen brauchbar, sofern sie erhebliche Strahlungsanteile in den genannten Wellenlängenbereichen emittieren. Die Expositionszeiten betragen in jedem Fall im allgemeinen 10 Sekunden bis 30 Minuten, vorzugsweise 2 bis 15 Minuten.

Alternativ kann man die Passivierungsschicht auch durch Bestrahlen des mit einer aktivierten polymeren Zwischenschicht versehenen und in die Monomerenlösung getauchten Transducers erzeugen.

Je nach der Konzentration der Monomerenlösung, den Beschichtungsbedingungen (Auftragsmethode, Tauchbestrahlung), der Strahlungsintensität und der Bestrahlungsdauer erzeugt man durch die Pfropfung man eine fest haftende Sperrschicht aus dem Hydrogel, die im allgemeinen 10 nm bis 500 µm stark ist.

### Einbau der Biokomponenten

Da die Biokomponenten in der Regel empfindliche Moleküle sind, erfolgt ihr Einbau zweckmäßig nach dem Aufbringen der Sperrschicht aus dem organischen Hydrogel auf deren Oberfläche. Zweckmäßig wird die Biokomponente kovalent oder durch ionische Bindung mit der Sperrschicht verbunden. Im einzelnen kommt es auf die Natur der Biokomponente an. Wenn sie z.B. freie primäre oder sekundäre Aminogruppen enthält, was häufig der Fall ist, kann man sie ionisch mit Ammoniumstruktur oder kovalent über eine Carbonamidbrücke anbinden, letzteres z.B. in bekannter Weise mittels N-Hydroxysuccinimid. Man erreicht auf diese Weise eine dichte, die Meßempfindlichkeit steigernde und zugleich stabile und abriebbeständige Belegung der Oberfläche der Sperrschicht mit der Biokomponente.

### Verwendung des Biosensors

Der erfindungsgemäße Biosensor eignet sich, wie entsprechende Biosensoren nach dem Stand der Technik, Bestimmung eines Biomoleküls (oder Analyten) in einer wäßrigen Matrix, das (bzw. der) von der Biokomponente des Sensors spezifisch gebunden wird. So kann man z.B. Antikörper von HIV-Viren im menschlichen Blut detektieren, um eine Infektion von Spenderblut nachzuweisen.

## Patentansprüche

1. Biosensor mit einer immobilisierten Biokomponente zur spezifischen Bindung eines Biomoleküls (Analyten), das in einer wäßrigen Matrix vorliegt, gekennzeichnet durch eine Schicht aus einem organischen Hydrogel als Passivierungsschicht gegen unspezifische Bindungen.

2. Biosensor nach Anspruch 1, dadurch gekennzeichnet, daß das organische Hydrogel aus hydrophilen Vinylmonomeren besteht, die bei 20°C zu mindestens 1 Gew.-% in Wasser löslich sind.

3. Biosensor nach Anspruch 1, dadurch gekennzeichnet, daß das organische Hydrogel aus hydrophilen Vinylmonomeren besteht, die bei 20°C zu mindestens 10 Gew.-% in Wasser löslich sind.

4. Biosensor nach Anspruch 1, dadurch gekennzeichnet, daß das organische Hydrogel aus hydrophilen Vinylmonomeren besteht, die bei 20°C zu mindestens 40 Gew.-% in Wasser löslich sind.

5. Biosensor nach einem der Ansprüche 1 bis 4, dadurch gekennzeichnet, daß neben den hydrophilen Monomeren hydrophobe Vinylmonomere vorliegen.

6. Biosensor nach einem der Ansprüche 1 bis 5, dadurch gekennzeichnet, daß das organische Hydrogel bei der Bestimmung des Kontaktwinkels nach der Methode von R.J.Good et al. einen Wert von <40° zeigt.

7. Biosensor nach Anspruch 6, dadurch gekennzeichnet, daß der Kontaktwinkel <30° ist.

8. Biosensor nach einem der Ansprüche 1 bis 7, dadurch gekennzeichnet, daß das organische Hydrogel durch Pfropfpolymerisation des Polymers auf eine aktivierte polymere Zwischenschicht auf dem Transducer aufgebracht wird.

9. Biosensor nach einem der Ansprüche 1 bis 7, dadurch gekennzeichnet, daß das organische Hydrogel durch Pfropf(co)polymerisation der Monomeren, die das Hydrogel bilden, auf eine aktivierte polymere Zwischenschicht auf dem Transducer aufgebracht wird.

10. Biosensor nach einem der Ansprüche 1 bis 9, dadurch gekennzeichnet, daß die Biokomponente kovalent oder durch ionische Bindung mit der Passivierungsschicht verbunden wird.

11. Verwendung eines organischen Hydrogels als Passivierungsschicht bei der Herstellung von Biosensoren.

12. Verwendung des Biosensors nach einem der Ansprüche 1 bis 10 zur Bestimmung eines Biomoleküls (oder Analyten) in einer wäßrigen Matrix, das (bzw. der) von der Biokomponente des Sensors spezifisch gebunden wird.
